# EUROPEAN PATENT APPLICATION

(11) **EP 1 970 048 A1**
(43) Date of publication of application: **17.09.2008**
(21) Application number: 07251084.5
(22) Date of filing: 15.03.2007
(51) Int. Cl.: A61K 9/10, A61K 31/59, A61P 17/06

(54) **Polyaphron topical composition with vitamin D**

(71) Applicant: Drug Delivery Solutions Limited, Leatherhead, Surrey KT22 7RY (GB)
(72) Inventor: Wheeler, Derek Alfred, Leatherhead Surrey KT22 7RY (GB); Steele, David Fraser, Leatherhead Surrey KT22 7RY (GB)
(74) Representative: Hayes, Adrian Chetwynd

(57) **Abstract**

A topical composition comprising a continuous phase and at least one discontinuous phase, said composition comprising at least one polyaphron dispersion and at least one vitamin D or vitamin D analogue.

## Description

The present invention relates to a topical composition comprising at least one vitamin D or vitamin D analogue.

It is known for compositions comprising vitamin D or vitamin D analogues to be used for the treatment of a number of skin conditions.

For example, EP-B-474,517 discloses the use of compositions containing one or more 1α-hydroxylated-19-nor-vitamin D compounds with a triple bond in the side chain in the treatment of psoriasis.

US 4,871,723 discloses a process for treating psoriasis by topically applying a composition comprising vitamin D and a wax carrier. Specifically, US 4,871,723 discloses a composition comprising a) a pharmaceutically effective amount of an active-type vitamin D₃, b) a solvent selected from fatty acid esters, higher alcohols with 10 or more carbons and propylene carbonate and c) an oily carrier selected from white vaseline, yellow vaseline and liquid paraffin.

US 2005/002546 A1 discloses a pharmaceutical composition comprising an active vitamin D compound in emulsion pre-concentrate formulations, as well as emulsions and sub-micron droplet emulsions produced therefrom. In particular, the pharmaceutical compositions of US 2005/002546 A1 comprise
(a) a lipophilic phase component;
(b) one or more surfactants; and
(c) an active vitamin D compound.

The surfactant or surfactants are suitably present in an amount of from 1% to 90% by weight based on the total weight of the composition, and preferably from about 5% to about 85% by weight based on the total weight of the composition.

Presently available compositions contain relatively high concentrations of vitamin D or vitamin D analogues and surfactants which often lead to skin irritation and worsening of psoriasis. For example, in 1996 the Food and Drug Administration of America required that the label included in Dovonex (calcipotriene)- a product containing 0.005% calcipotriol - be amended to indicate that approximately 25% of patients experienced skin irritation, and approximately 10% worsening of psoriasis. In addition, it has been reported that some patients treated with Dovonex have developed hypercalcaemia (see, for example, Hardman KA, Heath DA, Nelson HM Hypercalcaemia associated with calcipotriol (Dovonex) treatment. BMJ. 1993 Apr 3;306(6882):896-896).

There is a need to formulate an improved composition suitable for topical application which addresses at least some of the problems of the prior art.

US-A-2006/0228408 discloses oral drug delivery systems comprising a biliquid foam which comprises a poorly water-soluble drug such as vitamin D. The compositions disclosed in US-A-2006/0228408 are suitable for oral delivery for which purpose the level of continuous phase (typically water) must be kept to a minimum.

The present inventors have now developed a new topical composition comprising at least one vitamin D or vitamin D analogue. The present inventors have surprisingly found that such compositions have an enhanced dermal diffusion rate and/or improved stability compared to known compositions. Such compositions also have an appropriate viscosity such that they are useful for topical application. The compositions also have an agreeable skin feel during and after topical application (which helps to ensure good patient compliance with the treatment regime).

Accordingly, the present invention provides a topical composition comprising a continuous phase and at least one discontinuous phase, said composition comprising at least one polyaphron dispersion and at least one vitamin D or vitamin D analogue.

According to another aspect of the present invention there is provided a composition as described herein for use in the treatment of psoriasis.

According to another aspect of the present invention there is provided a composition as described herein for use in the manufacture of a medicament for the treatment of psoriasis.

According to a further aspect the present invention provides a composition as described herein for use in a method of treatment of the human or animal body by therapy.

According to a further aspect there is provided a method of treatment or prophylaxis of psoriasis in a subject which comprises topically applying to a subject an effective amount of a composition as herein described.

In the following description, the meaning of the terms used are as follows: by hydrophilic phase or solvent is meant a liquid phase comprising water, comprising water together with other water-miscible liquids, or comprising a non-aqueous liquid which is miscible with water. By hydrophobic phase or solvent is meant a phase comprising pharmaceutically acceptable liquids such as oils that are immiscible or substantially immiscible with the hydrophilic phase. By immiscible liquids is meant that when mixed together, they separate to form two distinctly separate liquid phases sharing a well-defined interface. By substantially immiscible is meant that two liquids mixed as above having a well-defined interface between two phases where each phase may nevertheless contain small quantities of dissolved molecules of the other phase.

According to another aspect of the present invention there is provided a method of making the composition as described herein comprising the following steps:
(i) providing a hydrophilic solvent, optionally comprising at least one vitamin D or vitamin D analogue and/or a surfactant;
(ii) providing a hydrophobic solvent optionally comprising at least one vitamin D or vitamin D analogue and/or a surfactant;
(iii)mixing the hydrophilic solvent with the hydrophobic solvent under suitable conditions to form the composition comprising at least one polyaphron dispersion and at least one vitamin D or vitamin D analogue.

Advantageously, the compositions of the present invention have an enhanced dermal permeation of the active agent compared to known compositions. Thus, lower levels of vitamin D and vitamin D analogue may be required in the compositions of the present invention in order to achieve beneficial treatment results. As a result of having lower levels of vitamin D in the compositions of the present invention the likelihood of causing skin irritation is reduced.

A further particular advantage is that the compositions have good long term stability even at elevated temperature (40°C). Preferably, the compositions may contain water. This may be useful for dissolving water-soluble additives such as water-soluble preservatives, antioxidants, water-soluble permeation enhancers and the like.

A further advantage is that compositions of the present invention are typically manufactured at room temperature without the need to apply heat, making it less likely that actives will be damaged in the composition.

A yet further advantage of the composition of the present invention is that it need not comprise a high level of surfactant. In high concentrations surfactants are known to cause skin irritation. It is therefore desirable to keep the surfactant level to a minimum when applied to skin, and particular to damaged skin such as in case of psoriasis. Preferably the compositions of the present invention comprise less than 4% by weight of surfactant, more preferably less than 3%, more preferably still less than 2% by weight of the total composition.

By polyaphron dispersion as used herein is meant a particular kind of hydrophilic liquid-in-hydrophobic liquid or hydrophobic liquid-in-hydrophilic liquid dispersion comprising (a) a hydrophilic liquid miscible phase, (b) a second hydrophobic phase being immiscible or substantially immiscible with the first phase and (c) one or more surfactants, wherein the dispersed phase is in the form of small (e.g. micron to sub-micron diameter, but more usually at least 1 micron diameter) droplets, and the whole having the following characteristics, which distinguish polyaphron dispersions from conventional or common emulsions or other types of dispersion:
1. They are capable of existing in a stable form wherein the volume fraction of the dispersed phase (φᵢₚ) is greater than 0.7 and can be as high as 0.97. (φᵢₚ is the volume ratio of discontinuous to continuous phase expressed as a fraction).
2. The microscopic appearance of polyaphron dispersions where φip is greater than 0.7 is of an aggregate of individual droplets, pushed closely together into polyhedral shapes, resembling the appearance of a gas foam. In this form, the dispersion has gel-like properties and is referred to as a Gel Polyaphron Dispersion (GPD).
3. Stable polyaphron dispersions can be formed with a surfactant concentration less than 3% and more typically less than 2% by weight of the total composition.
4. Gel Polyaphron Dispersions (as described in 2 above) can be diluted to any extent by the addition of more continuous phase without the addition of more surfactant, when the gel-like properties disappear. Once φᵢₚ has been reduced to below 0.7, the individual droplets of internal phase become separated to take the form of spherical droplets, which remain stable and intact but which may nevertheless join together in loose associations and float to the top or sink to the bottom of the diluted dispersion (depending on the relative densities of the two phases). In this diluted form each droplet is referred to as a Colloidal Liquid Aphron (CLA). Simple shaking of the diluted dispersion instantly causes a homogeneous, stable dispersion of Colloidal Liquid Aphrons to re-form.

Each of the above characteristics and a combination of them clearly differentiate the polyaphron dispersions of the present invention from conventional emulsions, and other dispersion types which do not have all of those characteristics. Polyaphron dispersions are disclosed in the following literature references by Sebba: "Biliquid Foams", J. Colloid and Interface Science, 40 (1972) 468-474 and "The Behaviour of Minute Oil Droplets Encapsulated in a Water Film", Colloid Polymer Sciences, 257 (1979) 392-396, Hicks "Investigating the Generation, Characterisation, and Structure of Biliquid Foams", PhD Thesis, University of Bristol, 2005, Crutchley "The Encapsulation of Oils and Oil Soluble Substances Within Polymer Films", PhD Thesis, The University of Leeds, 2006 and Lye and Stuckey, Colloid and Surfaces, 131 (1998) 119-136. Aphrons are also disclosed in US-A-4,486,333 and WO 97/32559.

Polyaphron dispersions are sometimes referred to as 'Biliquid Foams', 'High Internal Phase Emulsions (HIPEs)', 'High Internal Phase Ratio Emulsions (HIPREs)' and 'Gel Emulsions'. All such descriptions that refer to dispersions having the characteristics described above are polyaphron dispersions of the present invention.

The present invention will now be further described. In the following passages different aspects of the invention are defined in more detail. Each aspect so defined may be combined with any other aspect or aspects unless clearly indicated to -the contrary. In particular any feature indicated as being preferred or advantageous may be combined with any other feature or features indicated as being preferred or advantageous.

As described above polyaphron dispersions comprise a continuous phase, a discontinuous phase and a surfactant. The discontinuous phase is preferably a substantially hydrophobic internal phase, commonly known as an oil internal phase. Preferably, the discontinuous phase comprises a pharmaceutically acceptable oil phase.

Examples of oils which may be used in the present invention include almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, isopropyl myristate, isopropyl isostearate, isopropyl palmitate, modified triglycerides, caprylic/capric glycerides, fractionated triglycerides, glyceryl tricaprate, glyceryl tricaproate, glyceryl tricaprylate, glyceryl tricaprylate/caprate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate, linoleic glycerides, saturated polyglycolized glycerides, synthetic medium chain triglyceride containing primarily C₈-C₁₂ fatty acid chains, medium chain triglycerides, long chain triglycerides, modified triglycerides, fractionated triglycerides, and mixtures thereof.

Suitably the discontinuous phase comprises monoglycerides, diglycerides or triglycerides.

It will be understood that other suitable oils may be used in the present invention.

The discontinuous phase may, for example, confer an emollient, occlusive, moisturising, conditioning or other cosmetic or pharmaceutical benefit to the skin. It may also increase the viscosity of the composition and may confer solvency to the active or actives.

The composition may comprise at least 1 % by weight of the discontinuous phase, preferably 1 to 80% by weight, more preferably from 10 to 80% by weight and most preferably from 25% to 80% by weight, based on the weight of the total composition.

The continuous or external phase of the polyaphron dispersion is preferably formed of water, hydrophilic liquid or a mixture thereof. The continuous phase preferably comprises from 20 to 99 wt%, more preferably from 20 to 75% and most preferably from 50 to 75% water based on the weight of total composition. When any part of the continuous phase is aqueous, close control of the pH within suitable limits is essential as would be understood by those skilled in the art.

The continuous phase may comprise or consist of water, a pharmaceutically acceptable liquid that is miscible or substantially miscible with water or a mixture thereof. The water miscible liquid is preferably a compound of formula R₁-OH where R₁ is C₁-C₁₀ alkyl and/or a compound of formula HO-R₂-H where R₂ is (C₂H₄)ₙ or (C₃H₆)ₙ where n is 1 to 100, preferably 1 to 25. R₁ and R₂ may be linear or branched. Preferably R₁ is C₁-C₄ alkyl. n is preferably 1 to 25. Preferably the continuous phase comprises water, propylene glycol or polypropylene glycol, polyethylene or polypropylene glycol, glycerol, ethanol, or a mixture thereof. Where the continuous phase comprises polyethylene glycol, the polyethylene glycol is preferably a polyethylene glycol which is liquid at room temperature. The polyethylene glycol may, for example, contain from 1 to 12 ethylene or propylene oxide units and/or have a molecular weight of up to 600.

It will be understood that other suitable hydrophilic solvents may be used in the continuous phase of the polyaphrons.

The surfactant used in the present invention may be incorporated into either or both phases of the polyaphron dispersion. Suitable surfactants include an alkyl polyglycol ether, an alkyl polyglycol ester, an ethoxylated alcohol, a polyoxyethylene sorbitan fatty acid ester, a polyoxyethylene fatty acid ester, an ionic or non-ionic surfactant, a hydrogenated castor oil/polyoxyethylene glycol adduct containing from 25 to 60 ethoxy groups a castor oil/polyoxyethylene glycol adduct containing from 25 to 45 ethoxy groups, a sorbitan fatty acid ester (for example Span 20 or Span 80), a block copolymer of ethylene oxide and propylene oxide (for example Pluronic L121 or Pluronic F68), or a mixture thereof.

It will be understood that other suitable surfactants may be used.

Preferably the compositions of the present invention comprise less than 4% by weight of surfactant, more preferably less than 3%, more preferably still less than 2% by weight of the total composition.

The composition of the present invention may comprise at least one vitamin D or vitamin D analogue predominantly in the continuous phase, or predominantly in the discontinuous phase. Most preferably at least one vitamin D or vitamin D analogue is present predominantly in the discontinuous phase.

The vitamin D analogue employed in the composition of the present invention may, for example, be calcipotriol, calcipotriol monohydrate, seocalcitol, calcitriol, calcipotriol hydrate, tacalcitol, maxacalcitol, paricalcitol, falecalcitriol, 1α,24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z),7(E),10(19)-triene, or a mixture thereof. More preferably, the vitamin D anolgue is calcipotriol, calcitriol, tacalcitol, maxacalcitol, 1α,24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z),7(E),10(19)-triene, or a mixture thereof. Most preferably, the vitamin D analogues are calcipotriol and calcipotriol hydrate. Other examples of suitable vitamin D analogues are described in US-B-6,753,013.

In one embodiment of the present invention, the vitamin D analogue is calcipotriol.

Synthetic vitamin D analogues are preferred in the compositions of the present invention over naturally occurring vitamin D or vitamin D derivatives, since the therapeutic effects of the latter may be less selective for the treatment of skin diseases, such as psoriasis.

The composition of the present invention may comprise from 0.0001 to 0.05% by weight of vitamin D or vitamin D analogue, preferably from 0.001 to 0.01% by weight and more preferably from 0.0025 to 0.005% by weight of the total composition.

Preferably, the composition of the present invention may further comprise a gelling agent and/or a viscosity modifying agent such as a rheology modifying agent

The gelling agent may, for example, be selected from alginate gums or their salts, guar gum, locust bean gum, xanthan gum, gum acacia, gelatin, hydroxymethyl-cellulose hydroxyethylcellulose, hydroxypropyl-cellulose, carboxymethylcellulose or its salts, bentonites, magnesium aluminium silicates, "Carbomers" (salts of cross-linked polymers of acrylic acid), or glyceryl polymethacrylates or their dispersions in glycols. It will be understood that other suitable gelling agents and/or a viscosity modifying agent or a rheology modifying agents could be used.

Preferably, the composition of the present invention comprises from 0.05 to 5.0% by weight of a gelling agent, preferably from 0.1 to 2.0% by weight and more preferably from 0.2 to 1.0% by weight of the total composition.

The composition of the present invention may be used in a method of treatment of the human or animal body by therapy. Further, the composition of the present invention may be used in the treatment of psoriasis. Also the composition of the present invention may be used in the manufacture of a medicament for treatment of psoriasis.

The compositions of the present invention may also contain other additives such as preservatives (for instance to prevent microbiological spoilage), buffering agents (for the control of pH and to avoid instability and damage to the skin's acid mantle), antioxidants and permeation enhancers. These additives may be included in the continuous or the discontinuous phase of the polyaphron dispersion.

It will be understood that the inclusion of these additives will be at the levels and with the type of materials which are found to be effective and useful. Care needs to be taken in the choice and amount of these additives to prevent compromise to the other performance advantages of the present invention.

The composition of the present invention may further comprise additional active agents, for examples, steroids or corticosteroids. Desirably, the composition does not comprise a corticosteroid.

In one embodiment of the present invention, the composition comprises at least one polyaphron dispersion, at least one vitamin D or vitamin D analogue and optionally at least one corticosteroid.

In a particularly preferred composition the discontinuous phase is a caprylic/capric triglyceride, the continuous phase is demineralised water and the vitamin D analogue is calcipotriol. This composition may optionally comprise or not comprise a corticosteroid such as betamethasone dipropionate

According to one aspect of the present invention, there is provided a method of making the composition as described herein comprising the following steps:
(i) providing a hydrophilic solvent, optionally comprising at least one vitamin D or vitamin D analogue and/or a surfactant;
(ii) providing a hydrophobic solvent optionally comprising at least one vitamin D or vitamin D analogue and/or a surfactant;
(iii) mixing the hydrophilic solvent with the hydrophobic solvent under suitable conditions to form the composition comprising at least one polyaphron dispersion and at least one vitamin D or vitamin D analogue.

In one embodiment of the present invention, the vitamin D or vitamin D analogue is mixed into the hydrophobic solvent prior to the polyaphron formation step. Alternatively, or additionally, the vitamin D or vitamin D analogue may be mixed into the continuous phase prior to the polyaphron formation step.

Suitable methods for preparing polyaphron dispersions are described in US-A-4486333. It will be understood by those skilled in the art that other manufacturing methods may be used, as appropriate.

The following Examples further illustrate the present invention.

### EXAMPLE 1

| ***Gel Polyaphron Oil Phase 1*** | **%w/w** |
|---|---|
| Solution comprising 0.00556% w/w calcipotriol, 1% Tween 80 in caprylic/capric triglyceride (Miglyol 812 - Condea) | 90.0 |
| | |

| ***Gel Polyaphron Aqueous Phase 1*** | |
|---|---|
| Poloxamer 188 (Pluronic F68 - BASF) | 1.0 |
| Demineralised Water | 9.0 |
| 50mM phosphate buffer | To pH 8.0 |

### Method of Manufacture of the Gel Polyaphron dispersion 1

A low form, 250ml laboratory beaker (internal diameter 6.5cm) was charged with sufficient aqueous (continuous) phase to make 30 g of gel polyaphron. This was stirred at 200 rpm with a four-bladed impeller having a diameter of 6.0 cm whilst adding the oil (discontinuous) phase dropwise from a Pasteur pipette. The rate of addition at the start of the process was slow (approximately one drop every 7 seconds) but was speeded up once 20% of the oil phase had been added so that the total time to make the gel polyaphron was approximately 20 minutes.

| ***Example 1*** | **%w/w** |
|---|---|
| Polyacrylic Acid Polymer (Ultrez 10 - Noveon) | 0.56 |
| Triethanolamine | To pH 7.5 |
| Demineralised Water | 55.04 |
| Gel Polyaphron 1 | 44.4 |

### Method of Manufacture of Example 1

The aqueous phase of the composition was made by pre-dispersing the Ultrez 10 into the water and then adding the triethanolamine until the pH had reached 7.7, by which time a clear gel had formed. The Gel Polyaphron dispersion 1 was then stirred into the gelled aqueous phase until homogeneously mixed.

Prior to the manufacture of each Gel Polyaphron dispersion, any active was dissolved in the appropriate phase by gentle stirring overnight with a magnetic stirrer at room temperature in a covered beaker.

### Stability Measurements

Stability measurements were made using the method outlined below.

The calcipotriol was extracted from the composition of Example 1 into isopropanol and assayed by HPLC under the conditions given below.

### HPLC conditions:

Column:NovaPakCl8, 4µ particle size, 3.9 x 150mm column (Waters)
Mobile phase: 47% acetronitrile in water.
Flow rate: 1ml/minute.
Column Temperature: 25°C.
Calcipotriol Retention Time: 6.9 minutes

The results show that after 3 months storage at 40°C, the level of calcipotriol was 99% ±1% of the original level.

### EXAMPLE 2

| ***Gel Polyaphron Oil Phase* 2** | **%w/w** |
|---|---|
| Solution comprising 0.00556% w/w calcipotriol, 1% laureth-4 in isopropyl myristate (IPM-NF - Inolex) | 90.0 |
| | |

| ***Gel Polyaphron Aqueous Phase 2*** | |
|---|---|
| Poloxamer 188 (Pluronic F68 - BASF) | 1.0 |
| Demineralised Water | 9.0 |
| 50mM phosphate buffer | To pH 8.0 |

### Method of Manufacture of the Gel Polyaphron dispersion 2

A low form, 250ml laboratory beaker (internal diameter 6.5cm) was charged with sufficient aqueous (continuous) phase to make 30 g of gel polyaphron. This was stirred at 200 rpm with a four-bladed impeller having a diameter of 6.0 cm whilst adding the oil (discontinuous) phase dropwise from a Pasteur pipette. The rate of addition at the start of the process was slow (approximately one drop every 7 seconds) but was speeded up once 20% of the oil phase had been added so that the total time to make the gel polyaphron was approximately 20 minutes.

| ***Example* 2** | **%w/w** |
|---|---|
| Polyacrylic Acid Polymer (Ultrez 10 - Noveon) | 0.56 |
| Triethanolamine | To pH 7.5 |
| Demineralised Water | 55.04 |
| Gel Polyaphron 2 | 44.4 |

### Method of Manufacture of Example 2

The aqueous phase of the composition was made by pre-dispersing the Ultrez 10 into the water and then adding the triethanolamine until the pH had reached 7.7, by which time a clear gel had formed. The Gel Polyaphron dispersion 2 was then stirred into the gelled aqueous phase until homogeneously mixed.

Prior to the manufacture of each Gel Polyaphron dispersion, any active was dissolved in the appropriate phase by gentle stirring overnight with a magnetic stirrer at room temperature in a covered beaker.

### EXAMPLE 3

The composition of Example 1 was tested for steady state of flux rate (measured over 12 hours) through silicone membranes in Franz diffusion cells for the calcipotriol. This is a recognised in vitro test correlating to permeation through the skin. For comparison the commercial products Dovonex and Dovobet were also tested.

| **Composition** | **Calcipotriol Flux Rate ng cm⁻² hr⁻¹** | **Ratio of Rate to that of Dovonex** |
|---|---|---|
| Example 1 Composition | 111.2 | 1.33 |
| Dovonex | 83.3 | 1.00 |
| Dovobet | 74.5 | 0.89 |

## Claims

1. A topical composition comprising a continuous phase, and at least one discontinuous phase, said composition comprising at least one polyaphron dispersion and at least one vitamin D or vitamin D analogue.

2. A composition according to claim 1 wherein the vitamin D or vitamin D analogue is vitamin D, calcipotriol, seocalcitol, calcitriol, tacalcitol, maxacalcitol, paricalcitol, falecalcitriol, 1α,24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z),7(E),10(19)-triene, or a mixture thereof.

3. A composition according to claim 2 wherein the vitamin D or vitamin D analogue is calcipotriol.

4. A composition according to any one of the preceding claims which comprises the vitamin D or vitamin D analogue in an amount of from 0.0001 to 0.05 wt% of the total composition.

5. A composition according to any one of the preceding claims wherein the discontinuous phase comprises an oil.

6. A composition according to claim 5 wherein the oil comprises a monoglyceride, diglyceride or triglyceride or a mixture thereof.

7. A composition according to any one of the preceding claims comprising at least 20% by weight of continuous phase based on the total weight of the composition.

8. A composition according to any one of the preceding claims wherein the continuous phase comprises from 20 to 99% by weight of water based on the total weight of the composition.

9. A composition according to claim 8 where the continuous phase comprises at least 25% by weight of water based on the total weight of the composition.

10. A composition according to any one of the preceding claims wherein the continuous phase comprises water, a compound of formula R₁-OH where R₁ is C₁-C₁₀ alkyl and/or a compound of formula HO-R₂-H where R₂ is (C₂H₄)ₙ or (C₃H₆)ₙ where n is 1 to 100 or a mixture thereof.

11. A composition according to claim 10 wherein the continuous phase comprises water, propylene glycol, glyercol, ethanol, or a mixture thereof.

12. A composition according to any one of the preceding claims wherein the vitamin D or vitamin D analogue is predominantly in the discontinuous phase.

13. A composition according to any one of the preceding claims further comprising a gelling agent.

14. A composition according to any one of the preceding claims further comprising a permeation enhancer.

15. A composition according to any one of the preceding claims which does not comprise a corticosteroid.

16. A composition as defined in any one of claims 1 to 15 for use in a method of treatment of the human or animal body by therapy, in particular by topical application.

17. A composition as defined in any one of claims 1 to 15 for use in the topical treatment of psoriasis.

18. Use of a composition as defined in any one of claims 1 to 15 in the manufacture of a medicament for the topical treatment of psoriasis.

19. A method of making the composition as defined in any one of claims 1 to 15 comprising the following steps:
(i) providing a hydrophilic solvent, optionally comprising at least one vitamin D or a vitamin D analogue, and/or a surfactant;
(ii) providing a hydrophobic solvent optionally comprising at least one vitamin D or a vitamin D analogue, and/or a surfactant;
(iii)mixing the hydrophilic solvent with the hydrophobic solvent under suitable conditions to form the composition comprising at least one polyaphron dispersion and at least one vitamin D or vitamin D analogue.
